# EUROPEAN PATENT SPECIFICATION

(11) **EP 2 465 543 B1**
(45) Date of publication and mention of the grant of the patent: **01.05.2013**
(21) Application number: 10195831.2
(22) Date of filing: 19.12.2010
(51) Int. Cl.: A61L 2/00, B82Y 30/00

(54) **Apparatus for sterilizing or disinfecting the hands of a person**
Vorrichtung zur Sterilisation und Desinfektion der Hände einer Person
Appareil pour stériliser ou désinfecter les mains d'une personne

(43) Date of publication of application: 20.06.2012
(73) Proprietor: Pastore, Marino, 8706 Meilen (CH); Pastore, Manuela, 8706 Meilen (CH); Agnoletto, Maria, 30010 Camponogara (IT); Rizzi, Luciano, 30010 Camponogara (IT)
(72) Inventor: Pastore, Marino, 8706 Meilen (CH); Pastore, Manuela, 8706 Meilen (CH); Agnoletto, Maria, 30010 Camponogara (IT); Rizzi, Luciano, 30010 Camponogara (IT)
(74) Representative: Schmauder & Partner AG Patent- & Markenanwälte VSP

(56) References cited:
- RO-B1- 120 808
- US-A1- 2010 078 574

## Description

### Field of the Invention

The present invention generally relates to an apparatus for sterilizing or disinfecting the hands of a person, particularly but not limited to destroying microbial load present on operators' hands, e.g. in food industry, hospitals, but also in other public sector domains such as public transportation stations and vehicles, shopping areas, amusement areas, swimming pools and spas.

### Background of the Invention

Devices for sterilizing or disinfecting the hands of a person are generally known and are based on a large variety of concepts. These include, for example, the application of particular gels, sprays or lotions containing appropriate chemical disinfectants. However, the level of decontamination achieved with such substances is not always sufficient because exposure is often superficial or too short. Moreover, such disinfectants may cause irritation or other undesirable effects on the users' skin.

Another type of sterilizing device is based on ultraviolet irradiation in an appropriate wavelength range. For example, US Patent Application no. 2003/0018373 A1 discloses an apparatus for sterilizing or disinfecting a region of tissue of a patient. This known device generally comprises a housing and an ultraviolet lamp disposed within the housing, and is optionally equipped with various additional components such as light seals and sensors for controlling the irradiation process.

### Summary of the Invention

In spite of many existing ultraviolet sterilizing devices, there is still a substantial need for improvement. In particular, it would be desirable to have a sterilizing or disinfecting apparatus that achieves a decontamination level of about 99% or better for bacterial microorganisms such as *Escherichia Coli, Pseudomonas Aeruginosa* and *Staphylococcus.* At the same time, the apparatus should be easy and safe to operate, and it should be compact, reliable and easy to handle and maintain. The above and other objects are achieved with the apparatus according to the present invention.

According to the present invention, there is provided an apparatus for sterilizing or disinfecting the hands of a person, the apparatus comprising:
- a housing provided with at least one entrance aperture for inserting the hands;
- ultraviolet light emitting means disposed within the housing and directed towards an irradiation zone within the housing;
the housing having interior walls provided with a reflective surface for reflecting ultraviolet light, said reflective surface being formed by a coating of titanium dioxide nanoparticles.

In general, the housing shall be of a stable material that does not transmit the ultraviolet light generated in its interior. Preferably, the housing is configured as a stainless steel box with two circular or near-circular apertures for hand insertion. For convenient operation, the housing will be mounted so as to have the apertures at an appropriate height for the intended users.

Ultraviolet light with a wavelength in the range of about 253 to 255 nm, particularly of about 253.7 nm, which is in the so called UV C band, may be conveniently generated with quartz lamps. Such UV light has turned out to have excellent germicidal effects towards bacteria, viruses, spores, fungi, mold and acari (including mites and ticks).

Surprisingly, it was found that the interior wall coating with titanium dioxide nanoparticles has synergistic effects in relation to the efficiency of the sterilizing or disinfecting process. On the one hand, the nanoparticles effectively give rise to an improved UV reflection and diffusion behavior, i.e. an optical improvement leading to uniform and thorough illumination of the user's hands. On the other hand, the nanoparticles provide a very large effective exchange area and thus exert a photo-catalytic effect. Accordingly, any microorganisms that happen to sit down or grow on the interior surface, for example during a period of non-operation or in case of strong microorganism exposure from outside, will be rapidly destroyed as soon as an illumination cycle is started.

Particularly advantageous embodiments are defined in the dependent claims.

In oder to have good and uniform UV illumination in the irradiation zone, it is advantageous to have the reflective surface cover substantially all of the interior walls. It is understood that some minor parts of the interior walls will be used for some special functions, e.g. for mounting the ultraviolet light emitting means or for mounting some sensor devices or the like. Nevertheless, the reflective surface should cover as much as possible, preferably at least 90 percent and even more preferably at least 95 perscent of the housing interior walls.

In order to achieve optimum UV reflection, it is preferable that said nanoparticles have an average grain size from 1 to 10 nanometers. Moreover, the coating will preferably have a thickness of 1 to 500, preferably 5 to 200 micrometers.

While it is understood that the ultraviolet light emitting means may have various configurations in principle, it appears particularly advantageous if the ultraviolet light emitting means comprise a pair of ultraviolet C sources directed at the irradiation zone, preferably from below and above, respectively. In this manner, it will be possible to have efficient UV coverage of the hand palms and backs.

In order to ensure optimum positioning of the hands and avoid any highly undesirable approach of the hands with the UV sources, it is particularly advantageous to define the irradiation zone by means of a birdcage mounted within the housing and having clearances matched to said entrance aperture.

In order to avoid any unwanted emergence of ultraviolet from the device during a sterilization or disinfection process, the entrance aperture is advantageously equipped with a light seal. Such a seal may be formed by a number of compliant flaps, e.g. made of a soft silicone material.

In a preferred embodiment, the apparatus further comprises means for controlling the execution of a predefined illumination cycle. In particular, these may comprise sensor means to detect the presence of hands, so as to start an illumination cycle when hands are inserted into the irradiation zone and for stopping the illumination cycle when hands are removed from the irradiation zone. Moreover, the apparatus may also comprise timer means for establishing a predefined duration of said illumination cycle, which will preferably last for less than a minute, preferably for 3 to 10 seconds and more preferably for about 4 to 6 seconds.

In order to improve compliance by the users, it will be advantageous if the apparatus according to claim 8, further comprises indicator means for displaying a status of said illumination cycle, for example "insert hands", "treatment" and "disinfection completed" to indicate the status before, during and shortly after an illumination cycle, respectively.

### Brief description of the drawings

The above mentioned and other features and objects of this invention and the manner of achieving them will become more apparent and this invention itself will be better understood by reference to the following description of various embodiments of this invention taken in conjunction with the accompanying drawings, wherein:
- Fig. 1: shows an embodiment of the apparatus for sterilizing or disinfecting the hands of a person, in an elevation view from the backside, with a part of the backwall removed;
- Fig. 2: shows the apparatus of Fig. 1, in a side elevation view;
- Fig. 3: shows the upper part of the apparatus of Fig. 1, in a front elevation view.

### Detailed description of the invention

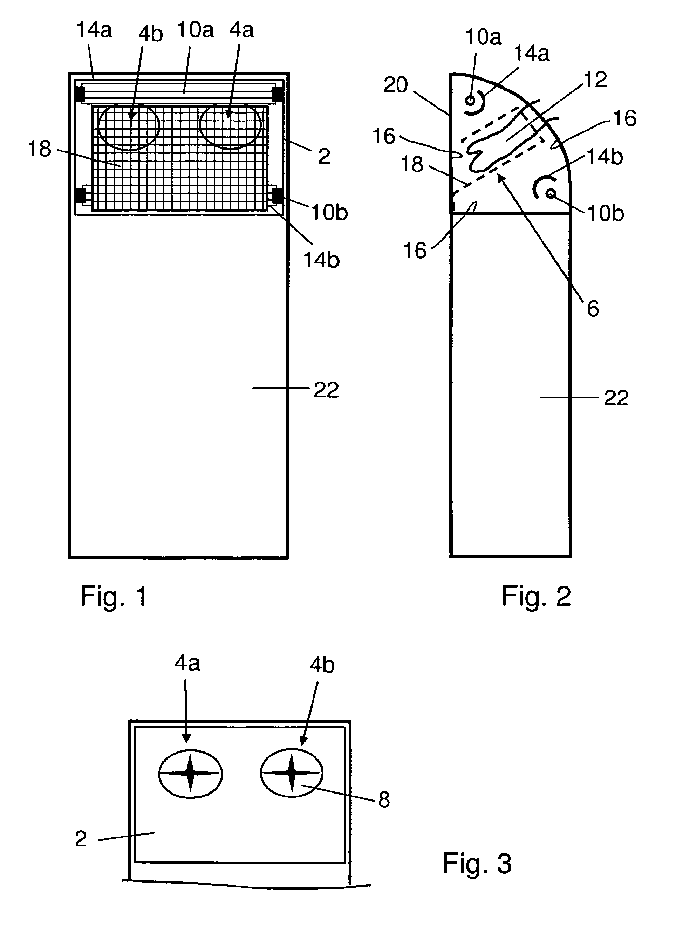

An apparatus for sterilizing or disinfecting the hands of a person is shown as an exemplary embodiment in Figs. 1 to 3. It comprises a housing 2, which, on the upper front side thereof, is provided with a pair of entrance apertures 4a and 4b for inserting the hands of a person into a central irradiation zone 6. Preferably, the housing is made of high quality stainless steel (e.g. AISI 304). In the example shown, each entrance aperture is provided with a plurality of flaps 8 acting as a light seal to prevent UV light from leaving the housing. Advantageously, the flaps 8 are made of a soft teflon-silicone material that allows for a comfortable passage of the hands.

Within the housing 2 there is a pair of tube-shaped quartz UV lamps 10a and 10b oriented essentially horizontally and mounted in an upper and lower region of the housing interior, respectively. In order to avoid direct irradiation of the person's hands 12, each UV lamp is provided with a light reflecting shield 14a, 14b, respectively, that is arranged between the lamp and the irradiation zone 6.

The interior walls of the housing 2 are provided with a reflective surface 16 that efficiently reflects the ultraviolet light originating from the UV lamps 10a, 10b and from the reflecting shields 14a, 14b. The reflective surface 16 is formed by a coating of titanium dioxide nanoparticles that creates a diffuse reflection pattern within the housing 2 and thus ensures homogenous irradiation of the hands 12 inserted therein.

In order to ensure proper placement of the person's hands 12, the irradiation zone 6 is configured as a birdcage 18 mounted within the housing 2 and having clearances matched to the entrance apertures 4a and 4b. It will be understood that the birdcage 18 may be mounted or locked to an inner surface of the housing 2 or it may simply rest on a bottom surface thereof.

In the present example, the housing 2 has a removable backwall panel 20, thus allowing for any necessary operations within the housing such as mounting and replacing the UV lamps 10a and 10b, positioning the birdcage 18, servicing the reflecting surface 16, and so forth.

Advantageously, the apparatus comprises sensor means for starting an illumination cycle when hands are inserted into the irradiation zone and for stopping the illumination cycle when hands are removed from the irradiation zone. Such sensor means, which are not shown in the drawings, may comprise an LED light source and an associated light detector arranged at opposite sides of the irradiation zone.

Moreover, the apparatus may comprise some type of timer means for establishing a predefined duration of said illumination cycle. This will avoid any UV damage to the hands if these are not withdrawn after an appropriate time. Advantageously, the apparatus will also comprise some type of indicator means for displaying the status of said illumination cycle, e.g. an indicator display showing "UV on" and "UV off", or some user instructions such as "Insert hands for disinfection", "Wait - Disinfection operation" and "Disinfection completed - Pull out hands".

It will be understood that the apparatus should be put in place in such manner that the entrance apertures 4a, 4b are easily accessible for the intended persons, particularly at an appropriate height. In the embodiment shown in the figures, the apparatus has a lower part 22 serving as a pedestal. In an alternative embodiment, the apparatus has no such lower part and thus is intended for placement on a table, sideboard or the like.

### Example

### Preparation of reflective surfaces

The reflective surfaces are prepared by coating with a suspension of titanium dioxide (TiO₂) anatase nanoparticles in water or glycol with a density from 1 to 1.2 g/ml. The (TiO₂) nanoparticles have a size in the range from a few nm up to 10 nm, which leads to a contact angle of about 5° to 11°. The suspension is applied with a spray or by painting on the inner walls of the housing with a permanent UV resistant coating. The coating thickness is between 1 and 500 micron.

### UV source

Suitable sources for the UV-C radiation are commercially available tube-shaped low pressure mercury lamps with a radiant power of 16 to 40 W, which can generate an irradiance at 1 meter distance of 40 µW/cm² to 127 µW/cm², respectively.

### Results

Experiments were carried out by inserting into the apparatus a series of Petri dishes containing the following culture media:
- Macconkey agar N.3 with *Escherichia coli* ATCC 25922,
- Mannitol Salt agar with *Staphylococcus aureus* ATCC 13150,
- Cetrimide agar with *Pseudomonas aeruginosa* ATCC 27853.

The samples were subjected to the UV-C irradiation protocol generally user for treating a person's hands. After an appropriate incubation time the number of Colony Forming Units per cm² (CFU/cm²) was evaluated for each Petri dish.

UV-C irradiation of *Escherichia coli* showed a reduction of the microbial load ranging from about 81%, where the starting load was at 4.38 CFU/cm², to about 92% where the initial loads were around 2.42 and 1.71 CFU/cm², respectively. UV-C irradiation of *Pseudomonas aeruginosa* gave a reduction of about 62% and 64% with starting loads of 5.08 and 1.30 CFU/cm².

UV-C irradiation of *Staphylococcus aureus* readily induced a reduction ranging from about 67% where the initial load was very high (8.11 CFU/cm²) to about 69% with a lower starting load of 5.25 CFU/cm².

### Concluding remarks

The above results demonstrate that with the present apparatus a UV-C irradiation of just 5 seconds is enough to substantially destroy the microbial load of *Escherichia coli, Staphylococcus aureus* and even *Pseudomonas aeruginosa,* which is notoriously very resistant to various disinfectants, on a person's hands. Accordingly, the apparatus for hands-sanitizing is a valid instrument for any situations in which hands hygiene plays an important role. The UV-C irradiation efficiently eliminates microorganisms on skin present after conventional water and soap cleaning without the need of chemical cleaners.

The apparatus is particularly useful for particular professional hygiene environments such as in food industry and hospitals, but also in the public sector (e.g. public transportation stations and vehicles, shopping areas, amusement areas, swimming pools and spas, etc.) where the importance of air quality is often underestimated and where the risk to catch illnesses, fungi, colds, flues etc. is enhanced.

### List of reference symbols

- 2: housing
- 4a, 4b: entrance aperture
- 6: irradiation zone
- 8: light seal
- 10a, 10b: UV light emitting means
- 12: hand
- 14a, 14b: reflecting shield
- 16: reflective surface
- 18: birdcage
- 20: backwall panel
- 22: lower part (pedestal)

## Claims

1. An apparatus for sterilizing or disinfecting the hands of a person, the apparatus comprising:
- a housing (2) provided with at least one entrance aperture (4a,4b) for inserting the hands (12);
- ultraviolet light emitting means (10a, 10b) disposed within the housing for irradiating an irradiation zone (6) located within the housing;
the housing having interior walls provided with a reflective surface (16) for reflecting ultraviolet light;
said reflective surface being formed by a coating of titanium dioxide nanoparticles.

2. The apparatus according to claim 1, wherein said reflective surface covers at least 90 percent of the housing interior walls.

3. The apparatus according to claim 1 or 2, wherein said nanoparticles have an average grain size from 1 to 10 nanometers.

4. The apparatus according to one of claims 1 to 3, wherein said coating has a thickness of 1 to 500 micrometers.

5. The apparatus according to one of claims 1 to 4, wherein said ultraviolet light emitting means comprises a plurality of tube-shaped quartz lamps (10a,10b), each lamp being provided with a light reflecting shield (14a,14b) arranged between the respective lamp and the irradiation zone (6).

6. The apparatus according to one of claims 1 to 5, wherein the irradiation zone is configured as a birdcage (18) mounted within the housing and having clearances matched to said entrance apertures (4a,4b).

7. The apparatus according to one of claims 1 to 6, wherein each one of said entrance apertures (4a,4b) comprises a light seal (8) to substantially prevent light from leaving the housing during sterilization or disinfection.

8. The apparatus according to one of claims 1 to 7, further comprising sensor means for starting an illumination cycle when hands (12) are inserted into the irradiation zone (6) and for stopping the illumination cycle when hands are removed from the irradiation zone.

9. The apparatus according to claim 8, further comprising timer means for establishing a predefined duration of said illumination cycle.

10. The apparatus according to claim 9, further comprising indicator means for displaying a status of said illumination cycle.

## Patentansprüche

1. Vorrichtung zur Sterilisation oder Desinfektion der Hände einer Person, wobei die Vorrichtung umfasst:
- ein mit mindestens einer Eintrittsöffnung (4a,4b) zum Einführen der Hände versehenes Gehäuse (2) (12);
- innerhalb des Gehäuse angeordnete UV-Licht emittierende Mittel (10a,10b) zur Bestrahlung einer innerhalb des Gehäuse angeordneten Bestrahlungszone (6);
wobei das Gehäuse mit einer reflektierenden Oberfläche (16) zur Reflexion von UV-Licht versehene Innenwände aufweist;
wobei die besagte reflektierende Oberfläche durch eine Beschichtung aus Titandioxid-Nanopartikel gebildet ist.

2. Vorrichtung nach Anspruch 1, wobei die besagte reflektierende Oberfläche mindestens 90 Prozent der Innenwände des Gehäuses bedeckt.

3. Vorrichtung nach Anspruch 1 oder 2, wobei die besagten Nanopartikel eine durchschnittliche Korngrösse von 1 bis 10 Nanometern haben.

4. Vorrichtung nach einem der Ansprüche 1 bis 3, wobei die besagte Beschichtung eine Dicke von 1 bis 500 Mikrometern hat.

5. Vorrichtung nach einem der Ansprüche 1 bis 4, wobei die besagten UV-Licht emittierenden Mittel eine Vielzahl von röhrenförmigen Quarzlampen (10a, 10b) umfassen, wobei jede Lampe mit einem zwischen der entsprechenden Lampe und der Bestrahlungszone (6) angeordneten lichtreflektierenden Schild versehen ist (14a,14b).

6. Vorrichtung nach einem der Ansprüche 1 bis 5, wobei die Bestrahlungszone als Vogelkäfig (18) ausgebildet ist, das innerhalb des Gehäuses befestigt ist und Durchgänge aufweist, die an besagte Eintrittsöffnungen (4a,4b) angepasst sind.

7. Vorrichtung nach einem der Ansprüche 1 bis 6, wobei jede der besagten Eintrittsöffnungen (4a,4b) eine Lichtabdichtung (8) umfasst, um den Austritt von Licht aus dem Gehäuse während der Sterilisation oder Desinfektion im Wesentlichen zu verhindern.

8. Vorrichtung nach einem der Ansprüche 1 bis 7, weiterhin umfassend Sensormittel zum Einschalten eines Beleuchtungszyklus wenn Hände (12) in die Bestrahlungszone (6) eingeführt werden und zum Abschalten des Beleuchtungszyklus wenn Hände aus der Bestrahlungszone entfernt werden.

9. Vorrichtung nach Anspruch 8, weiterhin umfassend Zeitmessmittel zum Einrichten einer vorbestimmten Dauer des besagten Beleuchtungszyklus.

10. Vorrichtung nach Anspruch 9, weiterhin umfassend Indikatorenmittel zum Anzeigen eines Status des besagten Beleuchtungszyklus.

## Revendications

1. Appareil pour stériliser ou pour désinfecter les mains d'une personne, cet appareil comprenant :
- un boitier (2) équipé d'au moins une ouverture d'entrée (4a, 4b) pour permettre d'introduire les mains (12),
- des moyens émettant de la lumière ultraviolette (10a, 10b) montés dans le boitier pour irradier une zone d'irradiation (6) située dans le boitier,
le boitier comportant des parois internes équipées d'une surface réfléchissante (16) permettant de réfléchir la lumière ultraviolette, cette surface réfléchissante étant formée par un revêtement de nanoparticules de dioxyde de titane.

2. Appareil conforme à la revendication 1, dans lequel la surface réfléchissante recouvre au moins 90 % des parois internes du boitier.

3. Appareil conforme à la revendication 1 ou 2, dans lequel les nanoparticules ont une granulométrie moyenne de 1 à 10 nanomètres.

4. Appareil conforme à l'une des revendications 1 à 3, dans lequel le revêtement a une épaisseur de 1 à 500 micromètres.

5. Appareil conforme à l'une des revendications 1 à 4, dans lequel les moyens émettant de la lumière ultraviolette comportent un ensemble de lampes à quartz en forme de tubes (10a, 10b), chacune de ces lampes étant équipée d'un écran réfléchissant la lumière (14a, 14b) situé entre la lampe respective et la zone d'irradiation (6).

6. Appareil conforme à l'une des revendications 1 à 5, dans lequel la zone d'irradiation est réalisée sous la forme d'une cage (18) montée dans le boitier et ayant des évidements adaptés aux ouvertures d'entrée (4a, 4b).

7. Appareil conforme à l'une des revendications 1 à 6, dans lequel chacune des ouvertures d'entrée (4a, 4b) comprend un élément assurant l'étanchéité à la lumière (8) pour empêcher essentiellement la lumière de quitter le boitier au cours de la stérilisation ou de la désinfection.

8. Appareil conforme à l'une des revendications 1 à 7, comprenant en outre des moyens de détection pour commencer un cycle d'éclairement lorsque des mains (12) sont introduites dans la zone d'irradiation (6) et pour stopper ce cycle d'éclairement lorsque les mains sont extraites de la zone d'irradiation.

9. Appareil conforme à la revendication 8, comprenant en outre des moyens d'horloge pour établir une durée prédéfinie du cycle d'éclairement.

10. Appareil conforme à la revendication 9, comprenant en outre des moyens d'indication pour afficher le statut du cycle d'éclairement.
